# EUROPEAN PATENT APPLICATION

(11) **EP 2 926 741 A1**
(43) Date of publication of application: **07.10.2015**
(21) Application number: 14166818.6
(22) Date of filing: 02.05.2014
(51) Int. Cl.: A61B 17/068, A61B 19/00

(54) **Adapter attachable to a shaft of an anchor delivery tool**

(30) Priority: 31.03.2014 US 201414229999
(71) Applicant: Coloplast A/S, 3050 Humlebaek (DK)
(72) Inventor: Taylor, Jeffrey Brian, Forest Lake, Minnesota 55025 (US)

(57) **Abstract**

A surgical system 30 configured to place an anchor 56 in tissue includes an anchor delivery tool 32 having a shaft 36 and an adapter 38 attachable to the shaft. The shaft provides a bore 52 and a length that are sized to retain a plurality of stacked anchors inside of the shaft. A rotatable anchor ejector 54 located inside of the bore of the shaft. The adapter has an segment 60 that is sized to be frictionally fit to an outside diameter of the shaft and a flange 62 that is connected to the segment. When the adapter is attached to the shaft, the flange extends distally beyond a distal end of the shaft.

## Description

### Background

Implanting a support material during surgery can present a challenge to the surgeon in that the surgeon is called upon to manipulate suturing or tacking instruments within relatively small spaces in the patient's body. In some cases, the surgeon is unable to see the target site and places the suture / tack by feel. In a laparoscopic surgery, the surgeon views the target site through a camera and manipulates tools and the patient's organs remotely through trocar access ports formed in the abdomen.

Support materials include hernia mesh that is useful in repairing perforations in the layers of the abdominal wall and organ support meshes. The hernia meshes are characterized as dense, durable, tear resistant and strong. Hernia meshes usually have a basis weight of more than 100 g/m². Hernia meshes usually have a small pore structure (it is tightly woven) that is designed to prevent or reduce tissue ingrowth through the hernia mesh. In contrast, organ support meshes are much less dense than hernia meshes and have relatively large openings (or pores) formed between the strands of the mesh, where the openings facilitate tissue ingrowth through the organ support mesh.

The tools that are employed to anchor hernia mesh material have been observed to be at times to be less than ideal for use in anchoring organ support meshes.

Improved instruments and methods of fixating implanted, lightweight organ support meshes would be welcomed by the surgical staff.

### Summary

One aspect provides a surgical system configured to place an anchor in tissue. The system includes an anchor delivery tool having a shaft and an adapter attachable to the shaft. A wall of the shaft provides a bore and a length that are sized to retain a plurality of stacked anchors inside of the shaft. A rotatable anchor ejector is located inside of the bore of the shaft. The adapter includes a segment that is sized to be frictionally fit to an outside diameter of the wall of the shaft and a flange that is connected to the segment. When the adapter is attached to the shaft, the flange has an inside flange diameter that is equal to or larger than a diameter of the bore and less than an outside diameter of the shaft to configure the flange to allow each of the plurality of stacked anchors inside of the shaft to exit the bore; and the flange extends distally beyond a distal end of the shaft.

### Brief Description of the Drawings

The accompanying drawings are included to provide a further understanding of embodiments and are incorporated in and constitute a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figure 1 is a perspective view of one embodiment of a kit of parts. The kit of parts includes a surgical system with an adapter that is attachable to a shaft of an anchor delivery tool.
Figure 2 is a perspective view of one embodiment of the adapter illustrated in Figure 1 apart from the shaft of the anchor delivery tool.
Figure 3 is a cross-sectional view of one embodiment of the adapter illustrated in Figure 1.
Figure 4 and Figure 5 are perspective views of the adapter illustrated in Figure 1 attached to the shaft of the anchor delivery tool.
Figure 6A is a cross-sectional view of the shaft of the anchor delivery tool illustrated in Figure 1 placed in contact with a mesh.
Figure 6B is a cross-sectional view of the adapter illustrated in Figure 1 attached to the shaft of the anchor delivery tool, with the adapter in contact with the mesh.
Figure 7 is a schematic view of the surgical system illustrated in Figure 1 employed to attach a pelvic organ prolapse mesh to a posterior wall of a vagina.
Figure 8 is a schematic view of the surgical system illustrated in Figure 1 employed to attach a pelvic organ prolapse mesh to an anterior wall of a vagina.
Figure 9 is a perspective view of one embodiment of a kit of parts. The kit of parts includes a surgical system with multiple adapters that are attachable to a shaft of an anchor delivery tool and a package of anchors.

### Detailed Description

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with reference to the orientation of the Figure(s) being described. Because components of embodiments can be positioned in a number of different orientations, the directional terminology is used for purposes of illustration and is in no way limiting. It is to be understood that other embodiments may be utilized and structural or logical changes may be made without departing from the scope of the present invention. The following detailed description, therefore, is not to be taken in a limiting sense, and the scope of the present invention is defined by the appended claims.

The features of the various exemplary embodiments described in this application are suitable and intended for combination with each other, unless specifically noted otherwise. An embodiment described earlier in the specification may be combined with an embodiment described later in the specification, and this is done to avoid needless repetition and redundancies.

Anterior means "forward" or "front," and posterior means "rearward" or "back." Relative to surfaces of an organ in the human body, an anterior surface will be oriented forward toward the belly and a posterior surface will be oriented rearward toward the spine.

End means an end most location and end portion means that segment adjacent to and near the end of an object. For example, two opposing ends of an object are each equidistant from a mid-point of the object and between the mid-point and each end of the object is an end portion of the object.

The Covidien ProTack™ Fixation Device is a market leading anchor fixation tool preferred by surgeons when implanting hernia mesh. The ProTack™ Fixation Device has a narrow annular shaft that is sized for laparoscopic access into the patient, where the shaft includes a rotatable anchor ejector retained within a bore of the shaft. The rotatable anchor ejector rotates to displace a helical titanium anchor out of the shaft and into soft tissue, which is useful when fixating hernia meshes. During a fixation procedure, the surgeon pushes the distal end of the shaft against the hernia mesh and activates a handle actuator to eject an anchor through the hernia mesh and into the tissue behind the hernia mesh. As noted above, hernia meshes are tightly woven to be dense, durable, tear resistant and strong with a basis weight of substantially more than 100 g/m². Thus, the fixation provided by the anchor of the ProTack™ Fixation Device is suitably strong and durable.

Unfortunately, the market leading anchor fixation tool faces challenges in fixating lightweight mesh in the treatment of pelvic organ prolapse. Restorelle® mesh provided by Coloplast, Minneapolis, MN uses Smartmesh® technology to provide a lightweight mesh that offers a more than 93% cure rate, and when implanted, maintains vaginal elasticity and cannot be felt by the patient's partner. Some Restorelle® meshes are lightweight meshes having a basis weight of between 10-50 g/m². Although the preferred choice for an anchoring tool, the rotatable anchor ejector of the Covidien ProTack™ Fixation Device has been observed to tear or otherwise damage the lightweight pelvic organ prolapse meshes during fixation.

Embodiments provide an adapter that is attachable to the shaft of an anchor delivery tool. The adapter provides a spacer between the mesh and the rotatable anchor ejector, which allows the anchor ejector to deploy the anchor from the shaft while preventing tearing or damage to the mesh from the ejector. Embodiments provide an adapter that converts the Covidien ProTack™ Fixation Device into an equally useful tool for fixating lightweight organ support meshes in both laparoscopic and open procedures.

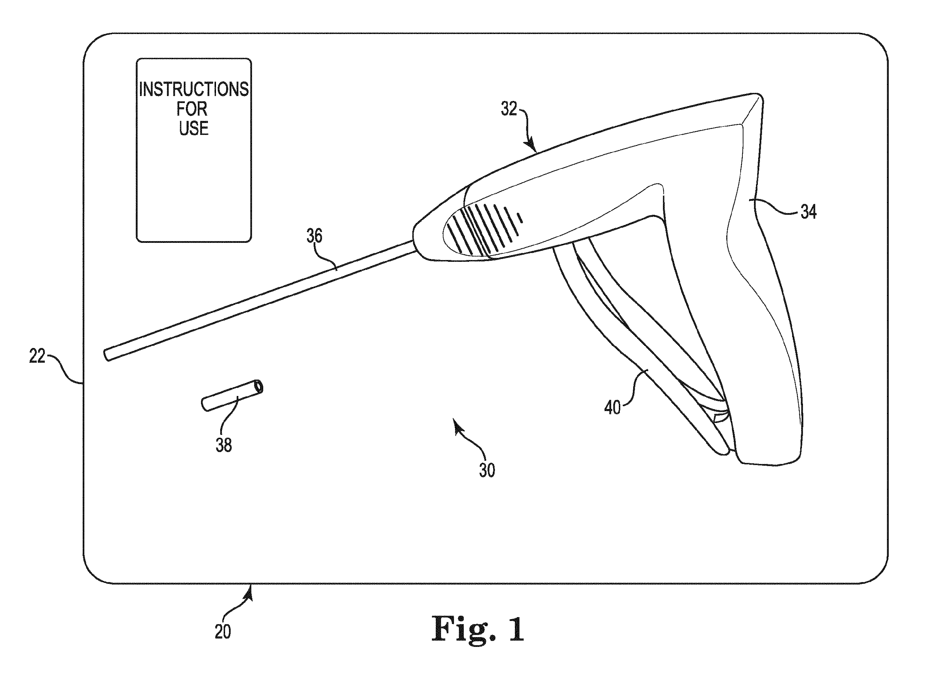

Figure 1 is a perspective view of one embodiment of a kit 20 of parts. The kit 20 of parts includes a package 22 containing a surgical system 30 and instructions for use of the system 30. The surgical system 30 is provided to fixate mesh to tissue and includes an anchor delivery tool 32 having an actuator handle 34 attached to a shaft 36, and an adapter 38 that is attachable to the shaft 36. One or more anchors are retained within the shaft 36. The kit 20 of parts suitably includes a package of anchors, where the anchors are stacked for delivery through the shaft 36. One useful configuration includes providing a package of six stacked, helical anchors for use with the system 30. One anchor is ejected out from the shaft 36 each time a trigger 40 of the actuator handle 34 is operated.

The kit of parts 20 is provided to a surgical facility as a single-use, sterile device that is useful in fixating mesh that is implanted into a patient's body. The anchor delivery tool 32 is suitable for use in open surgeries in which the shaft 36 is inserted directly into an incision formed in the patient's body, for example to access a target location located near a vaginal incision. The anchor delivery tool 32 is also suitable for use in laparoscopic surgeries in which the shaft 36 is directed down a cannula of the laparoscopic device for access to the internal organs of the patient. One suitable open surgery anchor delivery tool 32 is the StatTack™ Fixation Device available from Covidien, New Haven, CT. One suitable laparoscopic anchor delivery tool 32 is the ProTack™ Fixation Device available from Covidien, New Haven, CT.

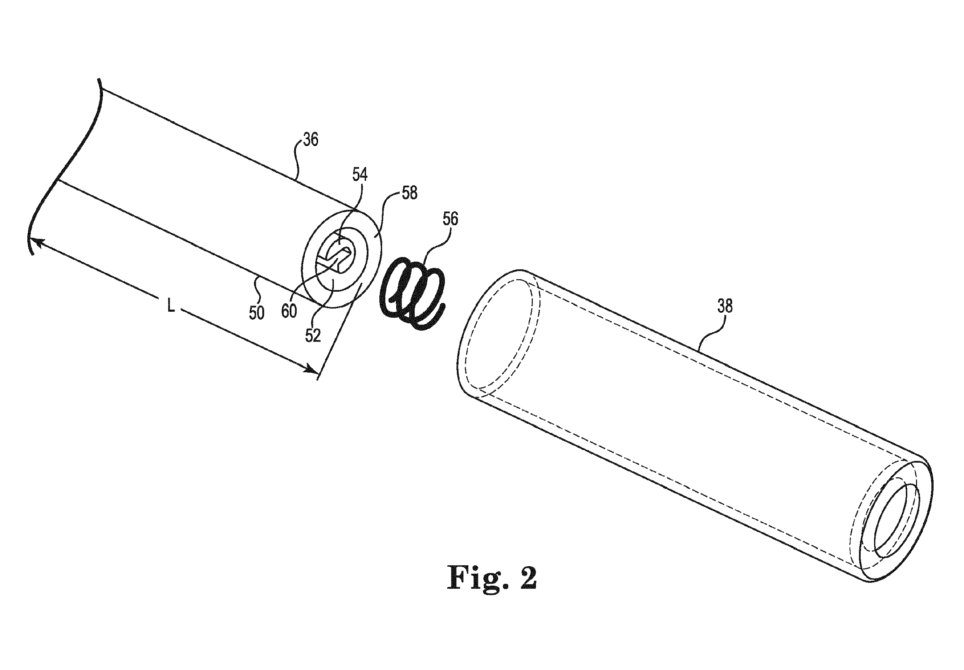

Figure 2 is a perspective view of one embodiment of the adapter 38 and an end of the shaft 36. The shaft 36 is provided as a thin-walled annular shaft having a length L. A wall 50 of the shaft 36 provides a bore 52. A rotatable anchor ejector 54 is retained within the bore 52 and is operable to eject an anchor 56 from the shaft 36 when the trigger 40 (Figure 1) is activated.

The rotatable anchor ejector 54 is located inside of the shaft 36 and is configured to rotate in one specific axial plane of the shaft 36. In one embodiment, the anchor ejector 54 is located at and restrained from moving from a distal end 58 of the shaft 36. The anchor ejector 54 generally rotates on a longitudinal axis of the shaft 36, and in so doing, unwinds or ejects the anchor 56 out of the shaft 36. In one embodiment, the anchor 56 is a helical anchor and the anchor ejector 54 includes a slot 60 that couples with an end of the helical anchor 56 to push the anchor 56 out of the shaft 36 as the anchor ejector 54 rotates.

Rotation of the anchor ejector 54 has the potential to snag or tear the implanted mesh material. Heavy, or high density hernia meshes are typically durable enough to resist the abrasion delivered by the anchor ejector 54. The modern, high-performance pelvic organ prolapse meshes that generally have a basis weight of less than 50 g/m² can be susceptible to tearing or damage from the rotation of the anchor ejector 54. The adapter 38 is provided for attachment to the shaft 36 to prevent or reduce contact of the anchor ejector 54 with the mesh that is being implanted.

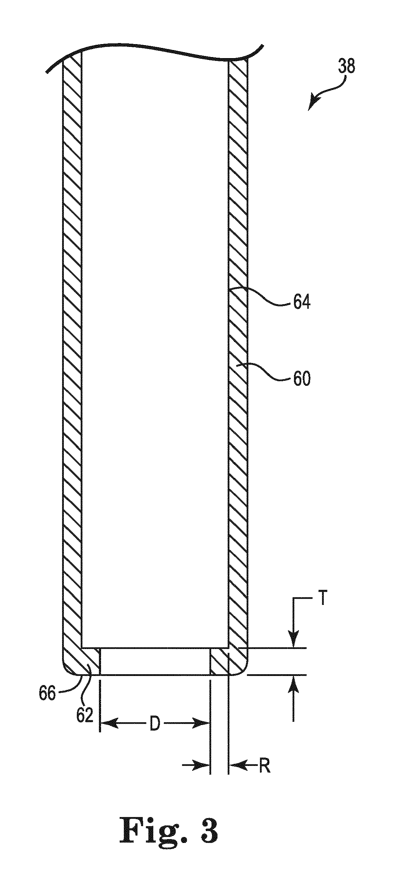

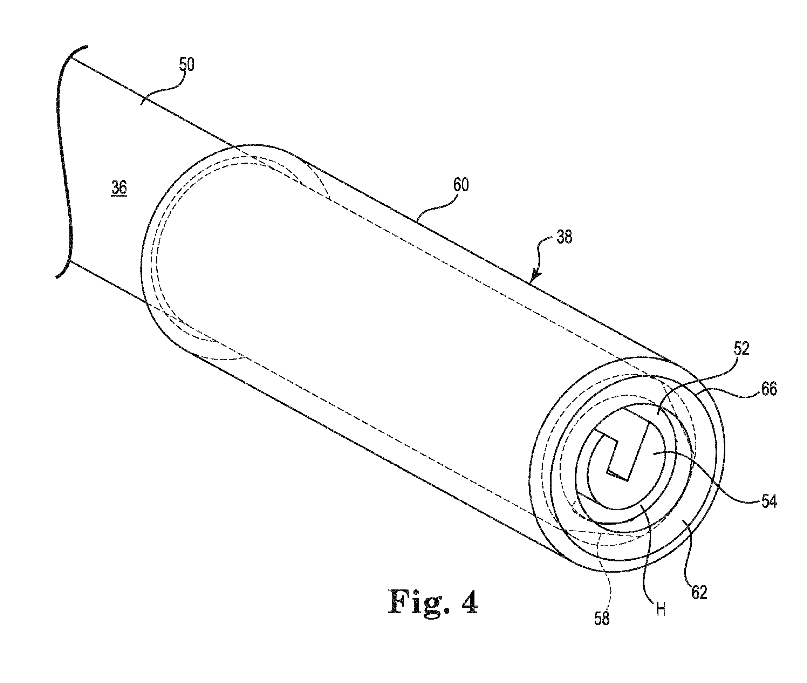

Figure 3 is a cross-sectional view of the adapter 38, and Figure 4 is a perspective view of the adapter 38 attached to the shaft 36. The adapter 38 includes an annular segment 60 and a flange 62. The annular segment 60 is sized to be frictionally fit over, or onto, an outside diameter of the annular shaft 36 and the flange 62 is connected to the annular segment 60. The flange 62 is sized to fit against the distal end 58 of the wall 50 of the shaft 36 while providing an opening H or a hole for the passage of anchors exiting the shaft 36. The hole H has a diameter D. The flange 62 extends a radial distance R from an inside surface 64 of the annular segment 60 to form the opening H in a distal end 66 of the adapter 38. The radial distance R is sized such that when the adapter 38 is attached to the shaft 36, the flange 62 will extend to the inside surface of the wall 50 of the shaft 36, which secures the adapter 38 to the shaft 36 and allows the anchors inside of the shaft 36 to exit the bore 52.

The diameter D is a flange diameter D. In one embodiment, the diameter D of the hole H is equal to or larger than a diameter of the bore 50 and less than an outside diameter of the annular shaft 36 to configure the flange 62 to provide clearance for anchors that exit the shaft 36. In one embodiment, the flange 62 has a flange thickness T that provides the adapter 38 with an off-setting spacer, such that when the adapter 38 is attached to the shaft 36, the flange 62 extends distally beyond the distal end 58 of the shaft 36. The flange thickness T or spacer dimension is selected to reduce or prevent the effects of the movement of the anchor ejector 54 against a surface of the mesh. The anchors 56 are ejected from the shaft 36 and the flange thickness T is selected such that a length of the anchor 56 is longer than the flange thickness T.

In one embodiment, the annular segment 60 of the adapter 38 is a sleeve (or cylindrical sleeve) that is sized to fit over a majority of a length of the annular shaft 36. A suitable range of length for the annular segment 60 is between 0.5 - 10 inches. One suitable length for the annular segment 60 is about 5 inches. One suitable range of thicknesses for the thickness T of the flange 62 is between 0.1 - 0.5 inches. One suitable thickness T for the flange 62 is about 0.25 inches.

The adapter 38 is suitably fabricated from plastic material or metal. Suitable plastic materials include polycarbonate, oriented polycarbonate, acrylonitrile butadiene styrene (ABS), polyolefins, melt-processable polymers, and non-melt-processable polymers referred to as curable polymer systems. Suitable fabrication processes for manufacturing the adapter 38 include machining, molding, or fabricating through stereo-lithography.

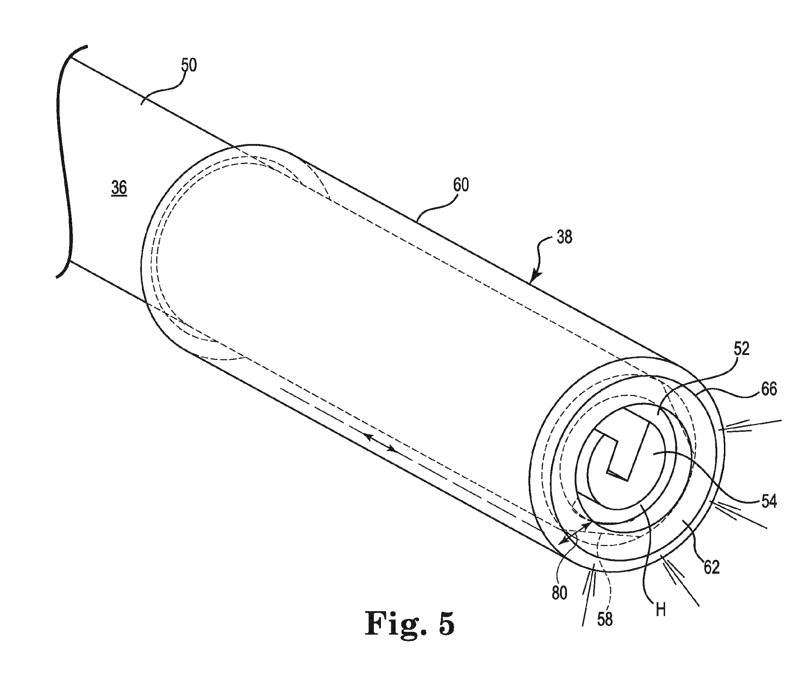

Figure 5 is a perspective view of one embodiment of the adapter 38 configured as a light pipe and attached to the shaft 36 of the anchor delivery tool 32. In one embodiment, the adapter 38 is fabricated from an oriented polycarbonate material in which the polymer chains in the annular segment 60 are oriented in a longitudinal alignment parallel to the longitudinal axis of the shaft 36. The flange 62 extends a radial direction away from the annular segment 60, and the polymer chains in the material are oriented in a radial direction. The flange 62 is illustrated extending at an angle of about 90 degrees relative to the annular segment 60, although acute angles of less than 90 degrees and obtuse angle of more than 90 degrees are also acceptable. The polymer chains in the flange 62 are approximately orthogonal to the longitudinal alignment of the polymer chains in the annular segment 60. A curved portion 80 is formed at the juncture where the flange 62 is attached to the annular segment 60, where the curved portion 80 forms a transition between the generally longitudinally aligned polymer chains in the annular segment 60 and the generally radially aligned polymer chains in the flange 62. An appropriate selection of the polycarbonate material configures the adapter 38 to provide a light pipe that transmits a light (originating in the laparoscopic system) along the adapter 38.

In one embodiment, the annular segment 60 and the flange 62 of the adapter 38 are monolithically formed of a material that is oriented to transmit visible light longitudinally along the annular segment 60 and also along the flange 62. The transmitted light becomes scattered at the curved portion 80 where the oriented polymer chains become concentrated in transition to the flange 62. The effect is that the curved portion 80 forms an annular band of scattered or diffuse light that is useful in illuminating the surgical field in the region of the flange 62. Thus, in one embodiment the adapter 38 provides a light pipe that produces a continuous halo of light in the region of the flange 62.

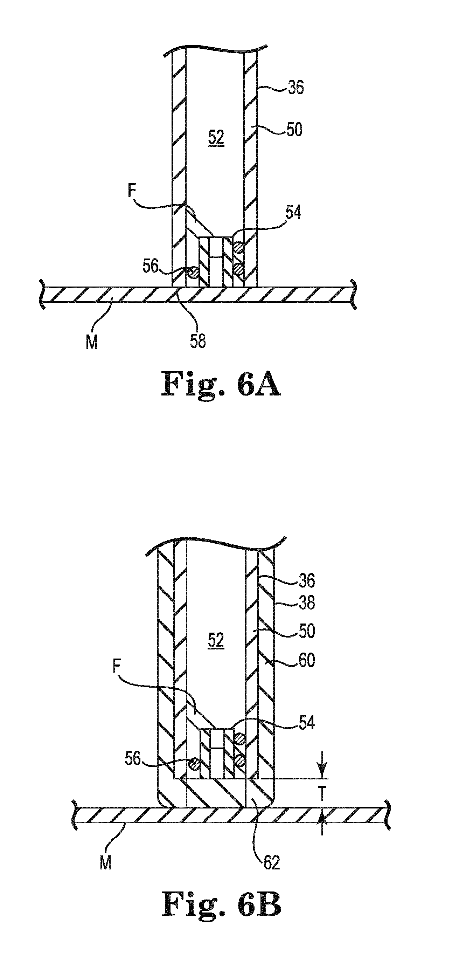

Figure 6A is a cross-sectional view of the distal end 58 of the shaft 36 pressed in contact with a surface of the mesh M. The anchor ejector 54 is located inside of the bore 52 of the shaft 36. The anchor 56 is engaged with the ejector 54 and with a helical flight F formed on the inside surface of the shaft 36. Rotation of the anchor ejector 54 rotates the anchor 56 along the helical flight F formed inside of the shaft 35, which operates to eject the anchor 56 out of the shaft 36 and through the mesh M. The anchor ejector 54 is retained within the plane of the distal end 58 of the shaft 36, and thus is in a position to contact the mesh M, especially as the shaft 36 is pressed forcefully against the mesh M. In cases where the mesh is a light weight mesh, for example with a basis weight of less than 50 g/m², and rotation of the anchor ejector 54 has been observed tear or otherwise damage the mesh M.

Figure 6B is a cross-sectional view of one embodiment of the adapter 38 connected to the shaft 36 of the anchor delivery tool 32 illustrated in Figure 1. The annular segment 60 of the adapter 38 is in frictional contact with the wall 50 of the shaft 36. The flange 62 of the adapter 38 bottoms out against and is in contact with the wall 50 at the distal end 58 of the shaft 36. The adapter 38 provides a spacer distance equal to the thickness T of the flange 62, where the spacer distance operates to offset the anchor ejector 54 away from the mesh M. Rotation of the anchor ejector 54 rotates the anchor 56 along the helical flight F to reject the anchor 56 out of the shaft 36. The flange 62 of the adapter 38 allows the anchor 54 to exit the shaft 36 while preventing the anchor ejector 54 from contacting / tearing the mesh M.

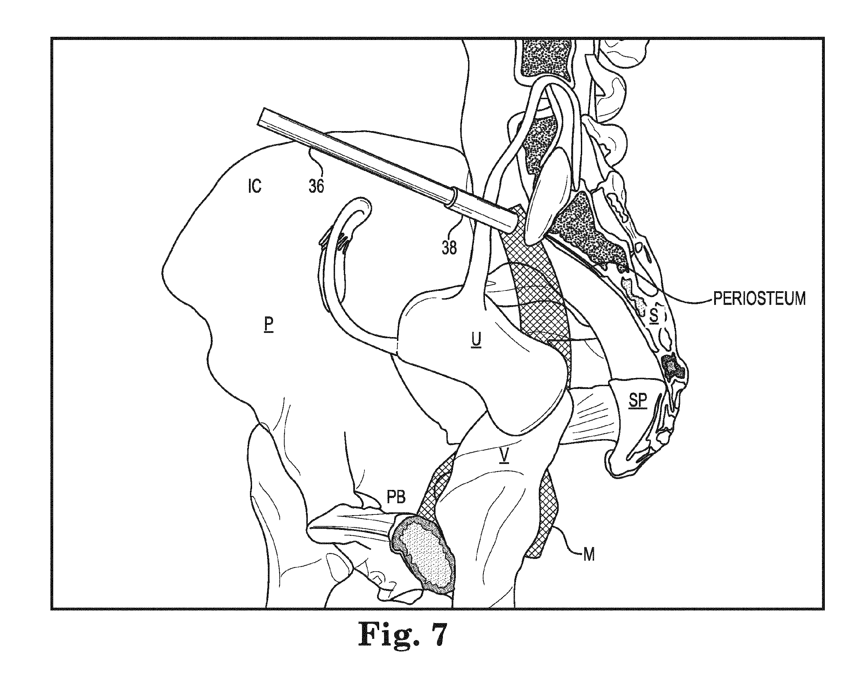

Figure 7 is a schematic view of a cross-section of a female pelvis P. The pelvis P is oriented with the pubic bone PB located anterior to the vagina V and the uterus U. The iliac crest IC is lateral to the pubic bone PB (behind the pubic bone PB in the image of Figure 7). The patient has not had a hysterectomy so the uterus U is attached to the vagina V. In a laparoscopic procedure, suitable laparoscopic ports are formed in the abdomen to provide access for the surgical tools to the interior of the pelvis P.

Figure 7 illustrates that the shaft 36 of the anchor delivery tool 32 (Figure 1) has been inserted through the abdomen for access to the sacrum S. The sacrum S is posterior of the uterus U and extends to the sacral promontory SP. The mesh M is attached between the posterior wall of the vagina V and the periosteum tissue that covers the sacrum S.

Mesh is useful in supporting the posterior wall of the vagina V in treatment of pelvic organ prolapse. One suitable mesh is a lightweight mesh sold by Coloplast Corp. under the brand Restorelle® mesh and is available from Coloplast Corp., Minneapolis, MN. The Restorelle® mesh are light weight meshes having a basis weight of between 10-50 g/m².

The mesh M is secured to the posterior wall the of vagina V and includes a tail portion that extends upward in a posterior direction to the sacrum S to provide support and elevation to the vagina V. In one procedure to treat pelvic organ prolapse, the adapter 38 is placed over the annular shaft 36 of the anchor delivery tool and inserted laparoscopically into the patient to attach the mesh M the periosteum tissue of the sacrum S. The surgeon will appropriately position the mesh M in the patient at the desired location relative to the sacrum S and subsequently fixate the mesh M to the periosteum tissue. The adapter 38 on the annular shaft 36 allows the surgeon to contact the mesh M with the adapter 38, maintain the mesh M in its desired location, and deliver an anchor through the mesh M and into the periosteum tissue to hold the tail of the mesh M in place. The handle actuator 34 (Figure 1) of the device 32 will be available outside of the patient to allow the surgeon to rotate the anchor ejector in the annular shaft 36 and eject one of the anchors 56 out of the shaft 36.

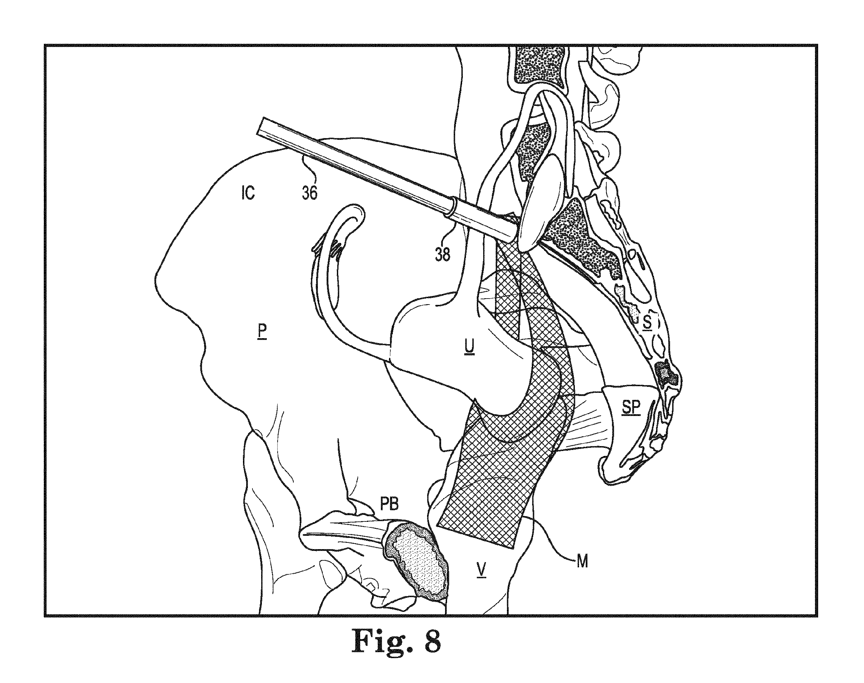

Figure 8 is a cross-sectional view of the pelvis P illustrating mesh M attached between the anterior wall of the vagina V and the sacrum S. The mesh M is suitably attached to the anterior wall of the vagina V and includes a first arm that extends around a first side of the uterus U and a second arm that extends around an opposite, second side of the uterus U. The arms diverge around the uterus U and are gathered at a posterior location and overlapped for attachment to the periosteum tissue over the sacrum S. The adapter 38 is placed on the shaft 36 of the anchor delivery device and inserted into the patient through a laparoscopic port. The surgeon maintains the arms at a desired location that elevates the anterior wall of the vagina V and subsequently fixates the arms of the mesh M to the sacrum S in a manner similar to that described above for fixation of the mesh to the posterior wall.

The implantation procedure of attaching the mesh in the patient includes the usual surgical steps of prepping the patient, suitably positioning the patient and sedating the patient, and forming an access port into the patient, either through a laparoscopic port or through an incision. Tissue is subsequently dissected to provide access to the area to be surgically repaired. The method of anchoring the mesh to tissue includes placing the adapter 38 onto the annular shaft 36 of the anchor delivery tool 32; inserting the annular shaft 36 and the adapter 38 into the patient; placing the mesh M into the patient at a desired location relative to the tissue; contacting the mesh M with the adapter 38 and maintaining the distal end 58 of the annular shaft 36 away from the mesh M; and rotating the anchor ejector 54 inside of the annular shaft 36 and ejecting an anchor 56 out of the annular shaft 36, out of the adapter 38, and through the mesh M and into the tissue. This method maintains the distal end of the annular shaft away from the mesh. This method achieves spacing the anchor ejector away from the mesh by a distance equal to the flange thickness.

Suitable additional steps for the method include laparoscopically inserting the annular shaft and the adapter into the patient; attaching a first portion of the mesh to a vagina of the patient and placing a second portion of the mesh against the sacrum of the patient; contacting the second portion of the mesh with the adapter and maintaining the distal end of the annular shaft away from the second portion of the mesh; and ejecting the anchor through the second portion of the mesh and into tissue of the sacrum.

Suitable additional steps for the method include laparoscopically inserting the annular shaft and the adapter into the patient; attaching a first mesh to a posterior wall of a vagina of the patient and attaching a second mesh to an anterior wall of the vagina of the patient; directing a tail of the first mesh around a uterus of the patient and directing a tail of the second mesh around the uterus of the patient; overlapping the tail of the first mesh over the tail of the second mesh and placing the tails of the first and second meshes against a sacrum of the patient; contacting one of the tails of the first and second meshes with the adapter and maintaining the distal end of the annular shaft away from the first and second meshes; and ejecting the anchor through the tails of the first and second meshes and into tissue of the sacrum.

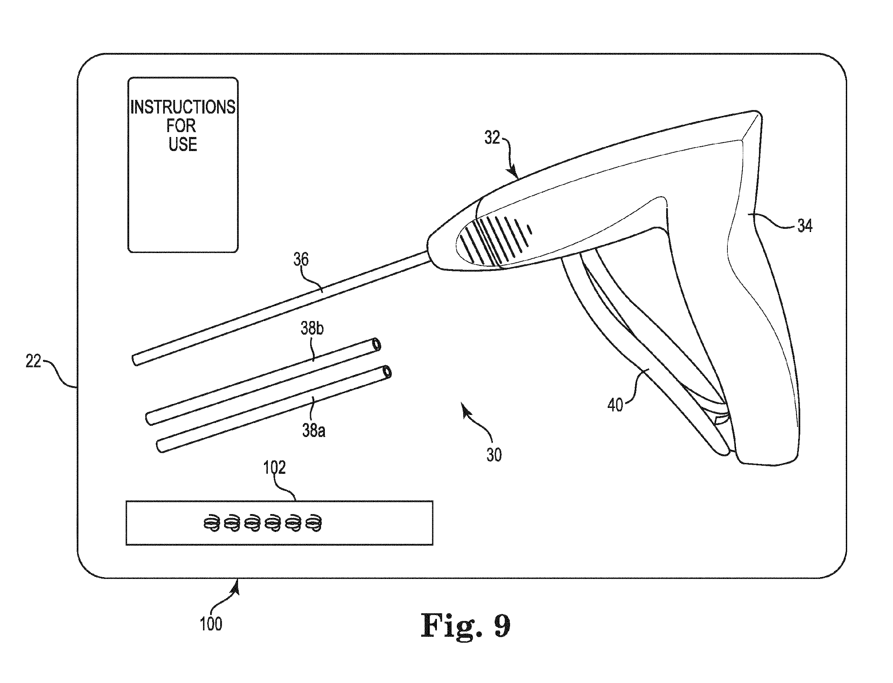

Figure 9 is a perspective view of one embodiment of a kit of parts 100. The kit of parts includes the surgical system 30 with multiple adapters 38a, b each attachable to the shaft 36 of the anchor delivery tool 32, a package 102 of anchors, and instructions for use of the system 30. The package 102 of anchors includes a package of stacked anchors for use in the anchor delivery tool 32. One useful package 102 of anchors is a package of stacked, titanium helical anchors for use in the anchor delivery tool 32. The adapters 38a, b have a similar diameter as the adapter 38 described above but are provided in a longer length of about 5 inches. The adapters 38a, b are configured to be slid in frictional engagement with the shaft 36. At least one of the adapters 38a, b is formed or configured to operate as a light pipe adapter and is fabricated from selectively oriented chains of light transmitting polymer.

Embodiments provide an adapter for an anchor delivery tool, where the adapter prevents the tool from undesirably tearing light weight meshes. The adapter provides a spacer between a working end of the tool and the mesh. Some embodiments of the adapter are provided as a light transmitting light pipe, which is useful when implanting meshes in hard to see areas of a patient. Embodiments provide an adapter that converts the Covidien ProTack™ Fixation Device into an equally useful tool for fixating lightweight organ support meshes in both laparoscopic and open procedures.

Although specific embodiments have been illustrated and described herein, it will be appreciated by those of ordinary skill in the art that a variety of alternate and/or equivalent implementations may be substituted for the specific embodiments shown and described without departing from the scope of the present invention. This application is intended to cover any adaptations or variations of medical devices as discussed herein. Therefore, it is intended that this invention is limited only by its claims and their equivalents.

## Claims

1. A surgical system configured to place an anchor in tissue, the system comprising:
an anchor delivery tool having a shaft with a wall of the shaft providing a bore that is sized to retain an anchor inside of the shaft, and a rotatable anchor ejector located in the bore of the shaft; and
an adapter attachable to the shaft, the adapter including a segment that is sized to be frictionally fit to an outside surface of the shaft and a flange that is connected to the segment;
wherein, when the adapter is attached to the shaft:
the flange has an inside flange diameter that is equal to or larger than a diameter of the bore and less than a diameter of the outside surface of the shaft to configure the flange to allow the anchor inside of the shaft to exit the bore; and
the flange contacts the wall and extends distally beyond a distal end of the shaft.

2. The surgical system of claim 1, wherein when the adapter is attached to the shaft, the flange extends a radial distance from the segment to an inside wall of the bore to provide clearance for the anchor inside of the shaft to exit the bore.

3. The surgical system of claim 1, wherein the anchor has an anchor length and the flange has a flange thickness that extends distally beyond the distal end of the shaft, and the anchor length is greater than the flange thickness.

4. The surgical system of claim 1, wherein the rotatable anchor ejector is restrained from moving distal to the distal end of the shaft.

5. The surgical system of claim 1, wherein the flange is configured to transmit light in a radial direction away from the segment of the adapter.

6. The surgical system of claim 1, wherein the adapter is a light pipe.

7. The surgical system of claim 1, wherein the segment and the flange of the adapter are monolithically formed of a material that is oriented to transmit visible light longitudinally along the segment and along the flange.

8. The surgical system of claim 1, wherein the segment of the adapter is an annular sleeve that is sized to fit over a majority of a length of the shaft.

9. A kit of parts comprising:
an anchor delivery tool, an adapter attachable to the anchor delivery tool, and instructions for use of the anchor delivery tool;
the anchor delivery tool including an annular shaft provided with a bore and a length that are sized to retain a plurality of stacked anchors inside of the annular shaft, and a rotatable anchor ejector located inside of the bore of the annular shaft; and
the adapter is sized to be frictionally fit to an outside surface of the annular shaft and includes a flange having an opening that is sized to allow each of the plurality of stacked anchors inside of the annular shaft to exit the bore, and the flanges forms a spacer that extends beyond a distal end of the annular shaft.

10. The kit of parts of claim 9, further comprising a package of stacked anchors.

11. The kit of parts of claim 9, further comprising a package of stacked helical anchors.

12. The kit of parts of claim 9, further comprising a plurality of adapters.

13. The kit of parts of claim 9, wherein the flange has an inside flange diameter that is larger than a diameter of the bore and less than an outside diameter of the annular shaft to configure the flange to allow each of the plurality of stacked anchors inside of the annular shaft to exit the bore.

14. The kit of parts of claim 9, wherein when the adapter is attached to the annular shaft, the flange extends a radial distance from the annular segment to an inside wall of the bore to provide clearance for each of the plurality of stacked anchors inside of the annular shaft to exit the bore.

15. The kit of parts of claim 9, wherein the adapter is a light pipe.

## Amended claims

### Amended claims in accordance with Rule 137(2) EPC.

1. A surgical system (30) configured to place an anchor (56) in tissue, the system comprising:
an anchor delivery tool (32) having a shaft (36) with a wall (50) of the shaft (36) providing a bore (52) that is sized to retain an anchor (56) inside of the shaft (36), and a rotatable anchor ejector (54) located in the bore (52) of the shaft (36); and
an adapter (38) attachable to the shaft (36), the adapter (38) including a segment (60) that is sized to be frictionally fit to an outside surface of the shaft (36) and a flange (62) that is connected to the segment (60);
wherein, when the adapter (38) is attached to the shaft (36):
the flange (62) has an inside flange diameter that is equal to or larger than a diameter of the bore (52) and less than a diameter of the outside surface of the shaft (36) to configure the flange (62) to allow the anchor (56) inside of the shaft (36) to exit the bore (52); andthe flange (62) contacts the wall (50) and extends distally beyond a distal end (58) of the shaft (36),
**characterized in that** the rotatable anchor ejector (54) is restrained from moving distal to the distal end (58) of the shaft (36).

2. The surgical system of claim 1, wherein when the adapter (38) is attached to the shaft (36), the flange (62) extends a radial distance from the segment (60) to an inside wall of the bore (52) to provide clearance for an anchor (56) inside of the shaft (36) to exit the bore (52).

3. The surgical system of claim 1, further comprising an anchor (56) having an anchor length and the flange (62) has a flange thickness that extends distally beyond the distal end (58) of the shaft (36), and the anchor length is greater than the flange thickness.

4. The surgical system of claim 1, wherein the flange (62) is configured to transmit light in a radial direction away from the segment (60) of the adapter (38).

5. The surgical system of claim 1, wherein the adapter (38) is a light pipe.

6. The surgical system of claim 1, wherein the segment (60) and the flange (62) of the adapter (38) are monolithically formed of a material that is oriented to transmit visible light longitudinally along the segment (60) and along the flange (62).

7. The surgical system of claim 1, wherein the segment (60) of the adapter (38) is an annular sleeve that is sized to fit over a majority of a length of the shaft (36).

8. A kit of parts (20) comprising:
an anchor delivery tool(24) and an adapter(38)according to claim 1, and instructions for use of the anchor delivery tool; wherein
the anchor delivery tool (24) including an annular shaft (36) provided with a bore (52) and a length that are sized to retain a plurality of stacked anchors inside of the annular shaft (36), and a rotatable anchor ejector (24) located inside of the bore (52) of the annular shaft (36); and the adapter (38) is sized to be frictionally fit to an outside surface of the annular shaft (36) and includes a flange (62) having an opening that is sized to allow each of the plurality of stacked anchors inside of the annular shaft to exit the bore (52), and the flange (62) forms a spacer that extends beyond a distal end (58) of the annular shaft (36).

9. The kit of parts of claim 8, further comprising a package (102) of stacked anchors.

10. The kit of parts of claim 8, further comprising a package (102) of stacked helical anchors.

11. The kit of parts of claim 8, further comprising a plurality of adapters (38).

12. The kit of parts of claim 8, wherein when the adapter (38) is attached to the annular shaft (36), the flange (62) extends a radial distance from the annular segment (60) to an inside wall of the bore (52) to provide clearance for each of the plurality of stacked anchors inside of the annular shaft (36) to exit the bore (52).

13. The kit of parts of claim 8, wherein the adapter (38) is a light pipe.
